# EUROPEAN PATENT SPECIFICATION

(11) **EP 1 744 808 B1**
(45) Date of publication and mention of the grant of the patent: **24.11.2010**
(21) Application number: 05735347.6
(22) Date of filing: 12.04.2005
(51) Int. Cl.: A61N 1/05

(54) **TRANS-SEPTAL CARDIAC PACING LEAD**
TRANS-SEPTALE KARDIALE ELEKTRODENLEITUNG
ELECTRODE DE STIMULATION CARDIAQUE TRANS-SEPTALE

(30) Priority: 27.04.2004 US 832738
(43) Date of publication of application: 24.01.2007
(73) Proprietor: Medtronic, Inc., Minneapolis, MN 55432-5604 (US)
(72) Inventor: STRUBLE, Chester, L., NL-6245 PH Ejisden (NL)
(74) Representative: Hughes, Andrea Michelle
(86) International application number: PCT/US2005/012238
(87) International publication number: WO 2005/107850

(56) References cited:
- WO-A-02/22206
- WO-A-02/094363
- US-A- 5 025 786
- US-A- 5 728 140
- US-A- 5 855 592
- US-A- 5 942 276
- US-B1- 6 241 728
- US-B1- 6 666 844

## Description

The present invention relates to implantable medical devices and more particularly to devices for pacing via a trans-septal approach.

Patients with poor atrio-ventricular conduction or poor sinus node function typically receive pacemaker implants to restore a normal heart rate. For another set of patients suffering from left bundle branch block (LBBB), left ventricular pacing and/or biventricular pacing has been shown to significantly improve cardiac hemodynamics and quality of life. However, some studies have shown that traditional pacing from a right ventricular (RV) apex can impair cardiac pumping performance. In some instances, ventricular wall abnormalities (ventricular remodeling) resulting from RV apical pacing have also been observed. So, alternative sites have been found where pacing can cause an electrical activation sequence similar to that in a normally activated heart and thus contribute to improved cardiac pump function.

From the literature there appear to be three major characteristics of normal cardiac electrical activation: 1.) Earlier activation of the left ventricle than right ventricle; 2.) Earlier endocardial activation than epicardial activation in left ventricular free wall; and 3.) Earlier activation in the apex than in the base of both ventricles. It has been found that a site of earliest activation occurs in the endocardium of the left ventricle along a lower portion of the inter-ventricular septum (i.e. near the apex) where it joins with the anterior wall of the heart. It would be desirable to pace at or near this site of earliest activation.

The present invention provides a system as defined in claim 1.

The following drawings are illustrative of particular embodiments of the invention and therefore do not limit its scope, but are presented to assist in providing a proper understanding of the invention. The drawings are not to scale (unless so stated) and are intended for use in conjunction with the explanations in the following detailed description. The present invention will hereinafter be described in conjunction with the appended drawings, wherein like numerals denote like elements, and:
A device according to the preamble of claim 1 is known from US 5 855 592.
Figure 1 is a schematic section through a heart wherein a pacing lead according to the system of claim 1 of the present invention is implanted;
Figure 2 is an enlarged view of a portion of Figure 1;
Figure 3 is a schematic section through a portion of a heart wherein a pacing lead according to the system of claim 1 of the present invention is implanted; and
Figure 4 is a schematic section through a portion of a heart wherein a delivery system according to an embodiment of the present invention is employed.

The following description is exemplary in nature and is not intended to limit the scope, applicability, or configuration of the invention in any way. Rather, the following description provides a practical illustration for implementing exemplary embodiments of the invention.

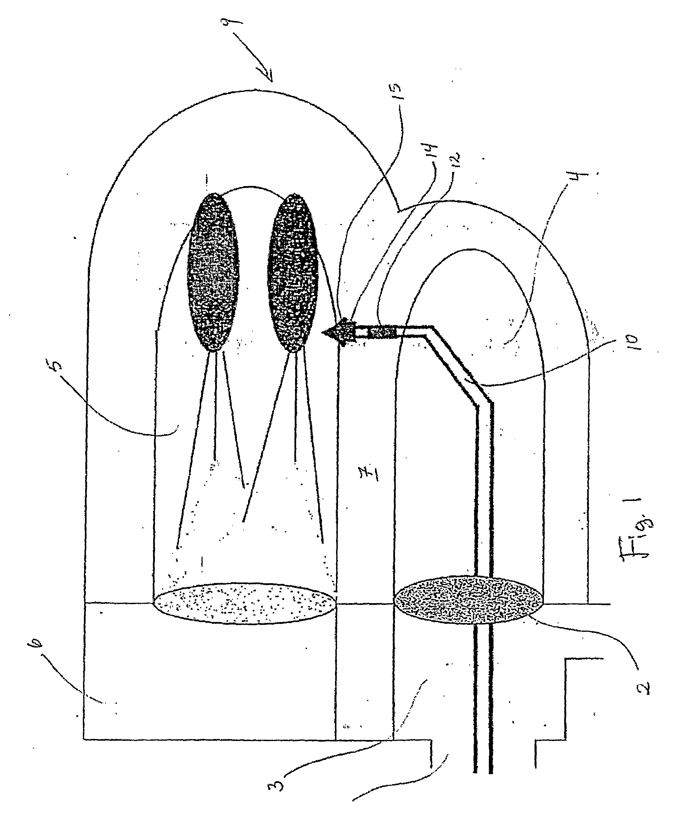

Figure 1 is a schematic section through a heart wherein a distal portion of a pacing lead 10 according to the system of claim 1 of the present invention is implanted. Figure 1 illustrates the distal portion of lead 10 extending through a superior vena cava 1, a right atrium 3 and a mitral valve 2 into a right ventricle 4; lead 10 includes an anode electrode 12 and a cathode electrode 14 which are shown implanted within an interventricular septal wall 7 in proximity to a left ventricular apex 9. Figure 1 further illustrates a tine-like structure 15 terminating a distal end of lead 10, which is within a left ventricle 5. It should be noted that lead 10 may be passed into the right heart via a standard transvenous route which may accessed by cephalic cut-down or subclavian stick; furthermore materials forming lead 10 and an arrangement of conductors, insulation and connector components may all conform to that of standard pacing leads. Tine-like structure 15, according to one embodiment, is formed of a resilient material allowing structure 15 to collapse as the distal portion of lead 10 is inserted through wall 7.

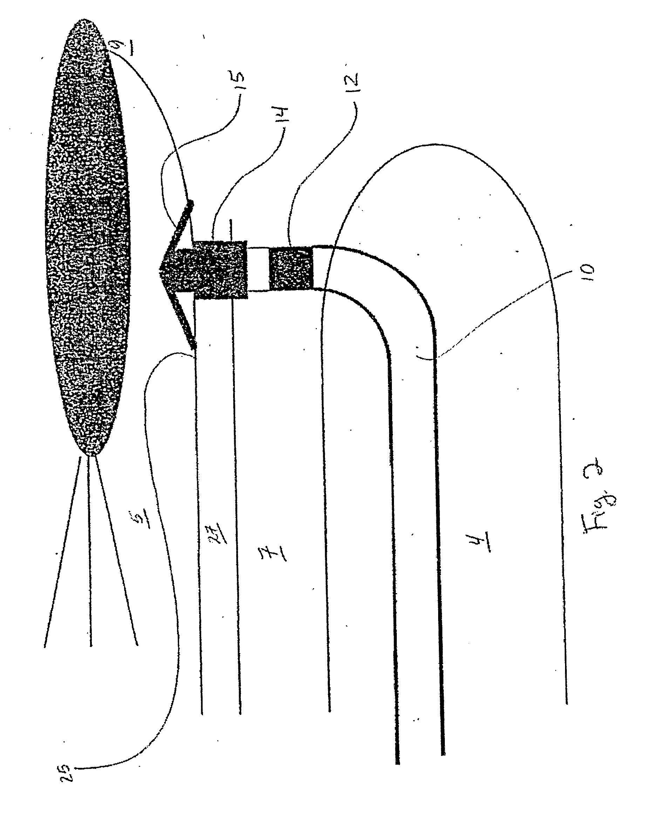

Figure 2 is an enlarged view of a portion of Figure 1 showing more specifically an implant site of cathode electrode 14 within a left ventricular endocardial layer 27 of septal wall 7. According to one embodiment of the present invention, the distal portion of lead 10 is inserted through septal wall 7 and then retracted to position electrodes 12 and 14, as illustrated, by means of feeling a resistance of structure 15 against a surface 25 of left ventricular endocardial layer 27; in this way structure 15 can serve as a depth gauge to assure that cathode electrode 14 is positioned for left ventricular endocardial pacing and sensing. It should be noted that alternative geometries of tine-like structures performing a similar function to structure 15, for example a hook geometry, can be incorporated into alternate embodiments of the present invention.

As is further illustrated in Figure 2, anode electrode 12 is spaced proximally from cathode electrode 14 so that when cathode 14 is positioned in left ventricular endocardial layer 27 anode 12 is positioned within a more central portion of septal wall 7. A thickness of septal wall 7, in proximity to left ventricular apex 9, may be between approximately 1.5 and approximately 2 cm, so that, according to some exemplary embodiments, a spacing between electrodes 14 and 12 is between approximately 5 mm and approximately 12 mm; state of the art electrode features including surface areas, macro and micro, and surface structure and treatments may be incorporated into some embodiments of the present invention. It should be noted that another embodiment of the present invention includes only electrode 14 and stimulation is unipolar, wherein a cardiac rhythm management device (not shown), to which lead 10 is coupled, serves as an indifferent electrode (- such devices and couplings are well known to those skilled in the art).

According to one embodiment which is not part of of the present invention, tine-like structure 15 is formed of a material adapted to dissolve in the blood soon after lead placement to reduce a risk for thrombus formation about structure 15. Examples of such materials include those taught in lines 10 - 24 of column 4 of U.S. Patent 6,173,206.

Figure 2 further illustrates cathode electrode 14 sized to serve as an anti-retraction feature, that is electrode 14 is oversized or includes an outer surface protruding radially from an adjacent portion of lead 10 just proximal to electrode 14.

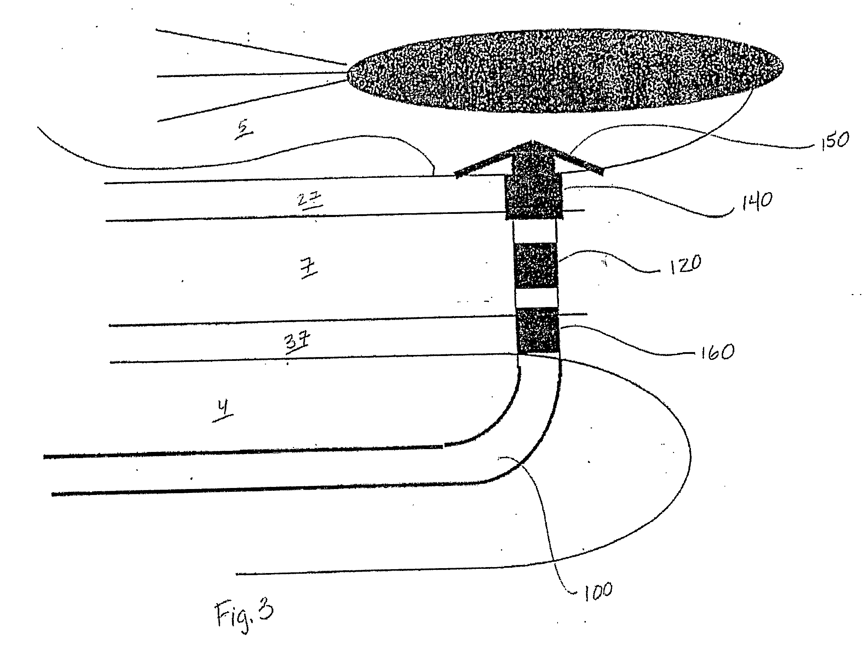

Figure 3 is a schematic section through a portion of a heart wherein a distal portion of a pacing lead 100 according to the system of claim 1 of the present invention is implanted; lead 100 extends into right ventricle 4 via a path very similar to that illustrated in Figure 1 for lead 10. Figure 3 illustrates lead 100 including a first electrode 140, a second electrode 120 and a third electrode 160. According to one embodiment which is not part of the invention first electrode 140 and third electrode 160 are each cathodes and second electrode 120 is an anode such that two bipolar pairs are formed for pacing and sensing, wherein first electrode 140 and second electrode 120 form a first bipolar pair for left ventricular pacing and sensing and third electrode 160 and second electrode 120 form a bipolar pair for right ventricular pacing and sensing. According to another embodiment which is not part of the invention, second electrode 120 is not included; in this case first electrode 140 and third electrode 160 are either operated in a unipolar mode or are adapted to alternate between polarities for bipolar operation such that, in one point in time, first electrode 140 is a cathode and third electrode 160 an anode for left ventricular pacing and sensing while, at another point in time, third electrode 160 is the cathode and first electrode 140 is the anode for right ventricular pacing and sensing.

According to some embodiments not part of the present invention, a pacing interval that appropriately times pacing pulses to right ventricular endocardium 37, via electrode 160, and left ventricular endocardium 27, via electrode 140, is programmed into a cardiac rhythm management device (not shown) to which lead 100 is coupled (- such devices and couplings are well known to those skilled in the art); preferably the interval is in sync with an innate electro-mechanical coupling between the electrode stimulation sites. Such an interval may be between approximately 0.5 milliseconds and approximately 100 milliseconds. Typically, in normal hearts, the natural conduction system activates the left ventricular endocardium prior to the right ventricular endocardium, so that according to one embodiment of the present invention, a pacing interval is set in which left ventricular pacing occurs prior to right ventricular pacing. According to some embodiments of the present invention, biphasic stimulation is incorporated, that is, a polarity for right ventricular pacing is the opposite of that for left ventricular pacing.

Figure 3 further illustrates lead 100 including a tine-like structure 150; according to one embodiment, structure 150 functions in a manner similar to structure 15 of lead 10 as previously described in conjunction with Figure 2. First electrode 140 is positioned with respect to structure 150, and second electrode 120 is positioned with respect to first electrode 140, and third electrode 160 is positioned with respect to second electrode 120, so that when lead 100 is implanted as illustrated, with structure 150 positioned in left ventricle 5, adjacent to endocardial surface 25, first electrode140 is located within left ventricular endocardium 27, second electrode 120 is located within a more central portion of septal wall 7 and third electrode 160 is located within a right ventricular endocardium 37. It should be noted that the scope of the present invention allows for spacings between electrodes (i.e. 12 and 14, 140 and 120, 120 and 160 and 140 and 160) that are not constrained to keep electrodes 14, 140 and 160 completely embedded in endocardial surfaces (i.e. 27 and 37), that is, portions of the cathode surfaces may protrude from the endocardial surfaces into the ventricles or may extend into a more central portion of the septal wall.

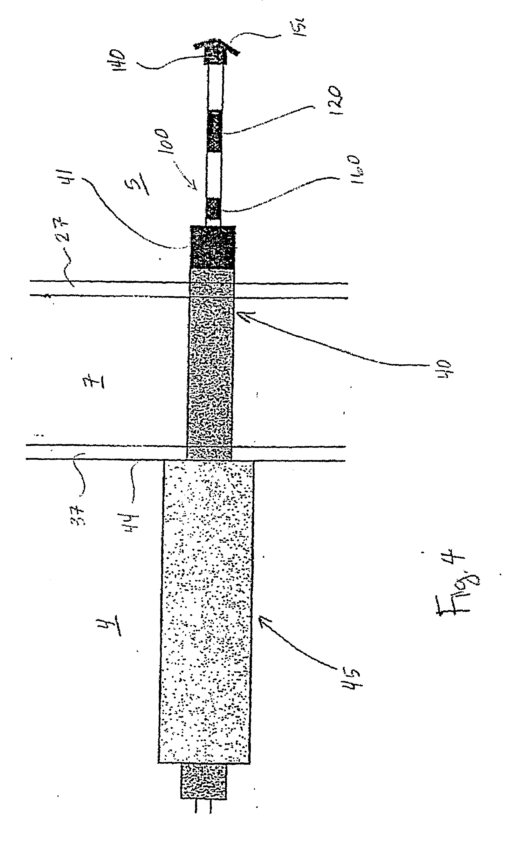

Figure 4 is a schematic section through a portion of a heart wherein a delivery system according to an embodiment not part of the present invention is employed. Figure 4 illustrates a distal portion of the delivery system, which includes a guiding catheter 45, a septal puncture needle 40 slideably received within the guiding catheter 45, and lead 100 slideably received within puncture needle 40. According to the illustrated embodiment, catheter 45 has been positioned against a surface 44 of right ventricular endocardium 37 so that needle 40, passing through catheter 45 may puncture through septal wall 7; guiding catheter 45 may be of a type of guiding catheter well known to those skilled in the art, which is constructed having a shape enabling positioning for a selected puncture site and a stiffness sufficient to provide backup support for puncturing. It may be determined via arterial blood backflow, from left ventricle 5 through needle 40, when needle 40 has punctured through wall 7; once the passageway is established by needle 40, lead 100 is passed through as illustrated. According to other embodiments of the present invention, an alternate method for passing lead 100 through wall includes first piercing through wall 7 with a tool to make a bore and then removing the tool to pass lead 100 through the bore. According to yet another embodiment, tine-like structure 150 includes a piercing tip so that lead 100, reinforced by an internal stiffening stylet may pierce through wall 7 without need for an independent piercing tool.

Figure 4 further illustrates an electrode 41 coupled to needle 40 in proximity to a distal end of needle 40 which may be used to sense and / or pace as needle 40 passes through septal wall 7. Once lead 100 has been passed through wall 7, as illustrated, needle 40 is pulled back out from wall 7 so that lead 100 may be retracted to position electrodes 140, 120, and 160 within wall, as illustrated in Figure 3.

Although embodiments of the present invention have been described herein in the context of cardiac pacing, it should be appreciated that embodiments of the present invention may be used for electrical stimulation of any body including a septum wherein it would be desirable to enter the septum from one side and pass through the septum to another side in order to position an electrode at or near that other side. Furthermore it may be appreciated that various modifications and changes can be made to the various embodiments described herein without departing from the scope of the invention as set forth in the appended claims.

## Claims

1. A system for delivering pacing comprising a delivery catheter including a lumen, and a medical electrical trans-septal pacing lead, comprising:
a lead body (10) including a distal end;
a structure (15) comprising a time terminating the distal end; and
a distal electrode (12, 14) coupled to the lead body at a position proximal to the structure; and further comprising a second electrode (14, 12) coupled to the lead body at a position proximal to the distal electrode; **characterized by**
a needle (40) slidably receiveable by the delivery catheter lumen, said needle adapted to puncture through a septal wall (7) and being supported by the delivery catheter, and including a needle lumen; and
said trans-septal pacing lead (100) being slideably receiveable by the needle lumen.

## Patentansprüche

1. System zum Bereitstellen einer Schrittmachertherapie bzw. zur Abgabe einer Stimulation, das einen Abgabekatheter mit einem Lumen und eine medizinische elektrische transseptale Schrittmacherleitung aufweist, mit:
einem Leitungskörper (10) mit einem distalen Ende;
einer Struktur (15), die eine Zacke aufweist, die das distale Ende abschließt; und
einer distalen Elektrode (12, 14), die in einer zu der Struktur proximalen Position mit dem Leitungskörper gekoppelt ist; und des Weiteren mit einer zweiten Elektrode (14, 12), die in einer zu der distalen Elektrode proximalen Position mit dem Leitungskörper gekoppelt ist, **gekennzeichnet durch**
eine Nadel (40), die **durch** das Lumen des Abgabekatheters verschiebbar aufgenommen werden kann, wobei die Nadel dazu ausgelegt ist, eine Septumwand (7) zu durchstechen, und **durch** den Abgabekatheter unterstützt bzw. getragen ist, wobei sie ein Nadel-Lumen aufweist; und
die transseptale Schrittmacherleitung (100), die durch das Nadel-Lumen aufgenommen werden kann.

## Revendications

1. Système pour délivrer une stimulation comportant un cathéter d'administration incluant une lumière, et une dérivation de stimulation trans-septale électrique médicale, comportant :
un corps de dérivation (10) incluant une extrémité distale ;
une structure (15) comportant une dent terminant l'extrémité distale ; et
une électrode distale (12, 14) couplée au corps de dérivation à une position proche de la structure ; et comportant en outre une seconde électrode (14, 12) couplée au corps de dérivation à une position proche de l'électrode distale ;
**caractérisé par**
une aiguille (40) pouvant être reçue par glissement par la lumière de cathéter d'administration, ladite aiguille étant adaptée pour poncturer à travers une paroi septale (7) et
étant supportée par le cathéter d'administration, et incluant une lumière d'aiguille ; et
ladite dérivation de stimulation trans-septale (100) pouvant être reçue par glissement par la lumière de l'aiguille.
